# EUROPÄISCHE PATENTSCHRIFT

(11) **EP 1 479 360 B1**
(45) Veröffentlichungstag und Bekanntmachung des Hinweises auf die Patenterteilung: **21.06.2006**
(21) Anmeldenummer: 04009630.7
(22) Anmeldetag: 23.04.2004
(51) Int. Cl.: A61F 5/01

(54) **Gelenkorthese**
Orthopaedic brace
Attelle orthopédique

(30) Priorität: 09.05.2003 DE 10321117
(43) Veröffentlichungstag der Anmeldung: 24.11.2004
(73) Patentinhaber: Otto Bock HealthCare GmbH, 37115 Duderstadt (DE)
(72) Erfinder: Wagner, Helmut, 37115 Duderstadt (DE); von Ascheberg, Alexander, 37115 Duderstadt (DE)
(74) Vertreter: Lins, Edgar

(56) Entgegenhaltungen:
- DE-C- 563 009
- GB-A- 720 512
- GB-A- 2 235 245
- US-A- 4 237 873
- US-A- 4 508 111

## Beschreibung

Die Erfindung betrifft eine Gelenkorthese mit einem ersten Gelenkteil, einer mit dem ersten Gelenkteil verbundenen ersten Schiene, einem zweiten Gelenkteil, einer mit dem zweiten Gelenkteil verbundenen zweiten Schiene und einem Drehgelenkteil zur gelenkigen Verbindung der zweiten Schiene und der ersten Schiene, wobei die Schienen aus entgegengesetzten Richtungen und seitlich zueinander versetzt in das Gelenkteil ragen, mit ihren Enden nebeneinander im Drehgelenkteil angeordnet sind und um ein geringes Maß gegeneinander drehbar sind.

Derartige Gelenkorthesen dienen insbesondere als Knöchelgelenkorthesen zur Unterstützung des Gehens und des Stehens einer im Unterschenkel- und Fußbereich gehandikapten Person. Das Drehgelenkteil dient dabei zur Ermöglichung einer relativ kleinen Drehbewegung des ersten Gelenkteils relativ zum zweiten Gelenkteil.

Bei den bekannten Orthesen dieser Art werden die beiden Schienen fluchtend miteinander ausgerichtet und um eine gemeinsame Drehachse relativ zueinander drehbar ausgebildet, wobei in dem Drehgelenkteil die Begrenzung der relativen Drehbewegung vorgesehen werden muss. Bei einer Knöchelgelenkorthese kann eine Fußheberfunktion dadurch bewirkt werden, dass das Fußteil mittels einer Feder in eine Ausgangs-Endstellung gedrückt wird, die durch den Druck beim Aufsetzen der Ferse des Fußteils (erstes Gelenkteil) gegen die Federkraft verlassen wird, um so eine gewisse Abrollbewegung beim Gehen zu ermöglichen.

Das Fußteil und das Unterschenkelteil (zweites Gelenkteil) sind regelmäßig aus Kunststoff schalenförmig gebildet, um den Fuß und den Unterschenkel stützend U-förmig zu umgreifen. Für eine stabile Verbindung der Fußschiene und des Fußteils wird eine gewisse Länge benötigt, die bei der bekannten Konstruktion unterhalb des Drehgelenkteils vorgesehen werden muss. Darüber hinaus ist eine gewisse Länge der Verankerung der Fußschiene in dem Drehgetenkteil erforderlich. Dadurch wird eine gewisse Mindest-Bauhöhe für das Fußteil bis zur Drehgelenkachse benötigt. Aus kinematischen und ästhetischen Gründen wäre es jedoch vorteilhaft, den Aufbau des Fußteils mit einer geringeren Höhe zu ermöglichen.

Durch DE 563 009 ist eine Gelenkorthese der eingangs erwähnten Art bekannt, bei der ein einen Fuß des Orthesenträgers untergreifendes Sohlenteil mit einer nach oben ragenden Seitenschiene verbunden ist, die aus zwei Teilen besteht, die durch ein Gelenk miteinander verbunden sind. Das Gelenkteil umfasst beidseitig das obere Ende der unteren Teilschiene und das untere Ende der oberen Teilschiene, wobei das untere Ende der oberen Teilschiene drehbar an dem Gelenkteil gelagert ist. Das obere Ende der unteren Teilschiene ragt über das Gelenkteil nach oben hinaus, um so die Drehbewegung des oberen Gelenkteils nach vorn zu begrenzen, also die gestreckte Stellung der Seitenschiene zu definieren. Das Gelenkteil ist dabei mit großem Abstand von dem Sohlenteil angeordnet, um sicher beim Tragen oberhalb des oberen Schuhrandes zu liegen. Der durch das obere Ende der unteren Schiene gebildete Anschlag ist dadurch verstellbar, dass stärkere oder schwächere Leder- oder Metallzungen oder ggf. ein verschiebbarer Keil zwischen den Schienen eingesetzt wird.

GB 2 235 245 A offenbart ein polyzentrisches Kniegelenk, das aus zwei im gestreckten Zustand fluchtend untereinander angebrachten Drehgelenken besteht, die in einem Gelenkgehäuse ausgebildet sind. Das Gelenkgehäuse begrenzt die maximale Streckung der Gelenkschienen mit Hilfe eines T-förmigen Einsatzes zwischen den Seitenwänden des Gelenkgehäuses. Durch unterschiedliche Einsätze können unterschiedliche Streckwinkel, die mehr oder weniger von 180° abweisen können, ausgebildet werden.

Der Erfindung liegt die Aufgabe zugrunde, eine Gelenkorthese der eingangs erwähnten Art so auszubilden, dass eine niedrige Bauhöhe eines Gelenkteils erreichbar ist.

Zur Lösung dieser Aufgabe ist erfindungsgemäß eine Gelenkorthese der eingangs erwähnten Art dadurch gekennzeichnet, dass das Drehgelenkteil zwei seitlich zueinander versetzt und im Wesentlichen parallel angeordnete Kammern aufweist, die in entgegengesetzten Richtungen offen sind und in die die Enden der Schienen hineinragen und dass die zweite Schiene im Endbereich ihrer Kammer um eine Drehachse drehbar so gelagert ist, dass die Drehbewegung der zweiten Schiene relativ zum Drehgelenkteil durch die Kammer begrenzt ist.

Bei der erfindungsgemäßen Gelenkorthese weist das Drehgelenkteil somit zwei Kammern auf, die im Wesentlichen parallel zueinander ausgerichtet sind, wobei eine Schiene in ihre (nach unten offene) Kammer fest einsteckbar ist, während die andere Schiene in die nach hinten versetzt angeordnete und vorzugsweise nach oben offene Kammer um einen gewissen Winkel drehbar eingesetzt ist. Die Drehachse befindet sich dabei im Endbereich der die Schiene drehbar aufnehmenden Kammer.

Die für die Funktion der beiden Kammern benötigten Längen sind somit im Drehgelenkteil nebeneinander realisiert und nicht - wie bisher üblich - in Richtung der miteinander fluchtenden Schienen untereinander, sodass eine Länge der Kammern in der Konstruktionshöhe der erfindungsgemäßen Gelenkorthese eingespart werden kann. Bei einer Knöchelgelenkorthese ergibt sich eine günstige Krafteinleitung in das Fußteil, die dadurch, dass die Fußschiene in Fußrichtung nach vorn gegenüber der Unterschenkelschiene versetzt angeordnet ist, mehr zur Fußmitte hin erfolgt und somit den natürlichen Gegebenheiten besser entspricht.

Darüber hinaus ermöglicht die erfindungsgemäße Gelenkorthese eine sehr einfache, aber stabile Konstruktion, indem in die Kammer der drehbar eingesetzten Schiene ein die Schiene seitlich führendes und Anschläge für die Drehbewegung der Schiene aufweisendes Einsatzteil eingesetzt ist. Die seitliche Führung und die Anschläge können somit aus einem hierfür geeigneten Kunststoffmaterial, beispielsweise PTFE, gebildet sein, obwohl die Schienen und das Drehgelenkteil vorzugsweise aus Metall bestehen.

Wenn in einer besonders bevorzugten Ausführungsform der Erfindung das Einsatzteil auswechselbar in das Drehgelenkteil eingesetzt ist, kann durch bloße Auswechslung des Einsatzteils eine Anpassung an den jeweiligen Patienten vorgenommen werden, indem die Winkelstellung für die stehende Ausgangsposition und der maximale relative Drehwinkel zwischen Unterschenkelschiene und Fußschiene festgelegt werden.

In sehr einfacher Form lässt sich bei einer Knöchelgelenkorthese auch eine Fußheberfunktion mittels einer Feder realisieren, die vorzugsweise ein Federgummiband ist, das um die Unterschenkelschiene einerseits und einen pilzartigen Befestigungsknopf auf der Oberseite des Drehgelenkteils andererseits unter Spannung auf Zug geschlungen ist.

Die Erfindung soll im Folgenden anhand eines in der Zeichnung dargestellten Ausführungsbeispiels näher erläutert werden. Es zeigen:
- Figur 1: eine Seitenansicht eines Ausführungsbeispiels einer erfindungsgemäßen, als Knöchelgelenkorthese ausgebildeten Gelenkorthese,
- Figur 2: einen Schnitt durch das in dieser Ausführungsform vorgesehene Drehgelenkteil.

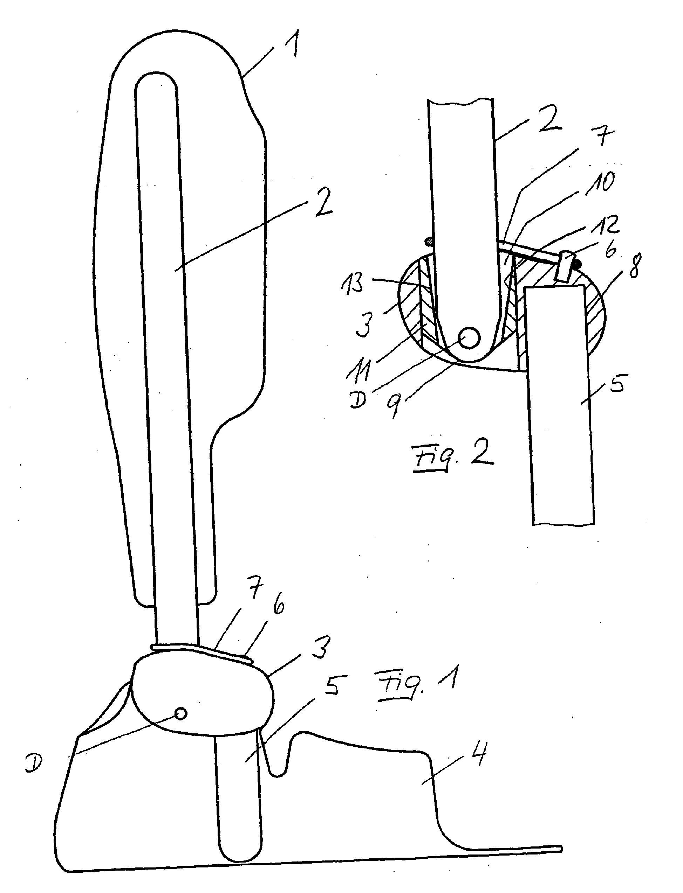

Die Ansicht der Figur 1 lässt ein schalenförmiges, stützendes Unterschenkelteil (zweites Gelenkteil) 1 erkennen, das zur Vorderseite hin offen ist, also den Unterschenkel seitlich um im Wadenbereich umgreift. Auf einer Seite ist das Unterschenkelteil 1 mit einer Unterschenkelschiene (zweiten Schiene) 2 fest verbunden. Die Unterschenkelschiene 2 ragt in ein Drehgelenkteil 3 hinein und ist in dem Drehgelenkteil 3 mittels einer Drehachse D drehbar gelagert. Seitlich nach vorn versetzt ragt in das Drehgelenkteil 3 von unten eine Fußschiene (erste Schiene) 5, die mit einem schalenförmigen, nach oben und vorn offenen Fußteil (erstes Gelenkteil) 4 verbunden ist. Die Fußschiene 5 ist in das Drehgelenkteil 3 fest, d. h. nicht seitlich bewegbar, eingesteckt.

Auf seiner Oberseite weist das Drehgelenkteil 3 einen pilzförmigen Befestigungsknopf 6 auf, um den ein Federgummiband 7 umläuft, das andererseits um die Unterschenkelschiene 2 geschlungen ist.

Die Schnittdarstellung der Figur 2 verdeutlicht, dass die Fußschiene 5 in eine nach unten offene, passende Kammer 8 des Drehgelenkteils 3 eingesteckt ist, sodass die Fußschiene 5 in dem Drehgelenkteil 3 nicht bewegbar ist.

Demgegenüber ragt die Unterschenkelschiene 2 mit einem vorderen, leicht verjüngend ausgebildeten und abgerundeten Ende 9 in eine nach oben offene Kammer 10 des Drehgelenkteils 3. In der Kammer befindet sich ein auswechselbar eingesetztes Einsatzteil 11 aus einem geeigneten, gut gleitenden Kunststoff, wie beispielsweise PTFE. Das Einsatzteil 11 führt die Unterschenkelschiene 2 seitlich für die Durchführung der Drehbewegung um die Drehachse D und legt mit einer vorderen Anschlagkante 12 und einer hinteren Anschlagkante 13 die Winkel für die Endpositionen der Drehbewegung der Unterschenkelschiene 2 relativ zu dem Drehgelenkteil 3 - und damit der Fußschiene 5 - fest. Durch Auswechslung des Einsatzteils 11 können somit bei -sonst unveränderten konstruktiven Teilen die Winkelstellung in der stehenden Position und der Drehwinkelbereich der Unterschenkelschiene 2 relativ zur Fußschiene 5 für den jeweiligen Patienten passend vorgegeben werden. Durch das auf Zug vorgespannte Federgummiband 7 wird das Fußteil 4 in der entlasteten Position relativ zum Unterschenkelteil 1 maximal nach oben gezogen, was einer Fußheberfunktion entspricht. Beim Aufsetzen des Fußteils 4 auf den Fersenbereich wird durch die Hebelwirkung des Fußteils 4 das Federgummiband 7 gelängt, sodass das Fußteil 4 gegenüber dem Unterschenkelteil 1 einen größeren Winkel einnimmt, um ein gewisses Abrollen des Fußteils 4 beim Gehen relativ zum Unterschenkelteil 1 zu ermöglichen.

Insbesondere Figur 2 verdeutlicht, dass die Drehachse D im unteren Bereich des Drehgelenkteils 3 angeordnet sein kann und dass die Fußschiene 5 in eine bis zum oberen Bereich des Drehgelenkteils 3 ragende Kammer 8 stabil einsetzbar ist, wodurch die Bauhöhe des Fußteils 4 bis zur Drehachse D deutlich reduziert werden kann, ohne Einbußen bei der Stabilität der Verankerung im Drehgelenkteil 3 hinnehmen zu müssen.

Es ist ohne Weiteres erkennbar, dass sich das in der Zeichnung dargestellte Ausführungsbeispiel mit entsprechenden Anpassungen auch als Ganzbeinorthese ausbilden lässt.

## Patentansprüche

1. Gelenkorthese mit einem ersten Gelenkteil (4), einer mit dem ersten Gelenkteil (4) verbundenen ersten Schiene (5), einem zweiten Gelenkteil (1), einer mit dem zweiten Gelenkteil (1) verbundenen zweiten Schiene (2) und einem Drehgelenkteil (3) zur gelenkigen Verbindung der zweiten Schiene (2) und der ersten Schiene (5), wobei die Schienen (2, 5) aus entgegengesetzten Richtungen und seitlich zueinander versetzt in das Gelenkteil (1) ragen, mit ihren Enden nebeneinander im Drehgelenkteil (3) angeordnet sind und um ein geringes Maß gegeneinander drehbar sind, **dadurch gekennzeichnet, dass** das Drehgelenkteil (3) zwei seitlich zueinander versetzt und im Wesentlichen parallel angeordnete Kammern (8, 10) aufweist, die in entgegengesetzten Richtungen offen sind und in die die Enden der Schienen (2, 5) hineinragen und dass die zweite Schiene (2) im Endbereich ihrer Kammer (10) um eine Drehachse (D) drehbar so gelagert ist, dass die Drehbewegung der zweiten Schiene (2) relativ zum Drehgelenkteil (3) durch die Kammer (10) begrenzt ist.

2. Gelenkorthese nach Anspruch 1, **dadurch gekennzeichnet, dass** die erste Schiene (5) in die ihr zugeordnete Kammer (8) fest eingesteckt ist.

3. Gelenkorthese nach Anspruch 1 oder 2, **dadurch gekennzeichnet, dass** in die Kammer (10) der zweiten Schiene (2) ein die zweite Schiene (2) seitlich führendes und Anschläge (12, 13) für die Drehbewegung der zweiten Schiene (2) aufweisendes Einsatzteil (11) eingesetzt ist.

4. Gelenkorthese nach Anspruch 3, **dadurch gekennzeichnet, dass** das Einsatzteil (11) auswechselbar in das Drehgelenkteil (3) eingesetzt ist.

5. Gelenkorthese nach einem der Ansprüche 1 bis 4, **dadurch gekennzeichnet, dass** die zweite Schiene (2) mittels einer Feder (7) gegen einen der Anschläge (12, 13) des Einsatzteils (11) drückbar ist.

6. Gelenkorthese nach Anspruch 5, **dadurch gekennzeichnet, dass** auf einer Oberseite des Drehgelenkteils (3) ein pilzartiger Befestigungsknopf (6) angeordnet ist, der von einem die zweite Schiene (2) umgebenden, auf Zug gespannten Federgummiband (7) umschlungen ist.

7. Gelenkorthese nach einem der Ansprüche 1 bis 6, **gekennzeichnet durch** ihre Ausbildung als Knöchelgelenkorthese mit einem Fußteil als erstes Gelenkteil (4) und einem Unterschenkelteil als zweites Gelenkteil (1)

## Claims

1. A joint orthosis with a first joint part (4), a first splint (5) connected to the first joint part (4), a second joint part (1), a second splint (2) connected to the second joint part (1), and a pivot hinge part (3) for articulated connection of the second splint (2) and of the first splint (5), said splints (2, 5) extending into the joint part (1) from opposite directions and laterally offset relative to one another, being arranged with their ends next to one another in the pivot hinge part (3), and being able to pivot to a small extent relative to one another, wherein the pivot hinge part (3) has two chambers (8, 10) which are arranged substantially parallel and are laterally offset relative to one another and which are open in opposite directions, and into which the ends of the splints (2, 5) extend, and wherein the second splint (2) is mounted in the end area of its chamber (10) so as to be able to pivot about a pivot axis (D) in such a way that the pivoting movement of the second splint (2) relative to the pivot hinge part (3) is limited by the chamber (10).

2. The joint orthosis as claimed in claim 1, wherein the first splint (5) is inserted fixedly into the chamber (8) assigned to it.

3. The joint orthosis as claimed in claim 1 or 2, wherein an insert part (11) is inserted into the chamber (10) of the second splint (2), which insert part (11) laterally guides the second splint (2) and has limit stops (12, 13) for the pivoting movement of the second splint (2).

4. The joint orthosis as claimed in claim 3, wherein the insert part (11) is inserted exchangeably into

5. The joint orthosis as claimed in one of claims 1 through 4, wherein the second splint (2) can be pressed by means of a spring (7) against one of the limit stops (12, 13) of the insert part (11).

6. The joint orthosis as claimed in claim 5, wherein a mushroom-shaped securing knob (6) is arranged on a top face of the pivot hinge part (3) and is looped by a tensioned resilient rubber band (7) surrounding the second splint (2).

7. The joint orthosis as claimed in one of claims 1 through 6, wherein it is designed as an ankle joint orthosis with a foot part as first joint part (4) and with a below-knee part as second joint part (1).

## Revendications

1. Articulation orthopédique comportant une première partie d'articulation (4), une première barre (5) reliée à la première partie d'articulation (4), une seconde partie d'articulation (1), une seconde barre (2) reliée à la seconde partie d'articulation (1), une partie d'articulation formant pivot (3) pour constituer une liaison articulée entre la seconde barre (2) et la première barre (5), les deux barres (2, 5) étant engagées dans la partie d'articulation (1) suivant des directions opposées et de façon décalée l'une à côté de l'autre, leurs extrémités étant disposées l'une à côté de l'autre dans la partie d'articulation formant pivot (3) et pouvant pivoter dans une mesure réduite l'une vers l'autre, **caractérisée en ce que** la partie d'articulation formant pivot (3) comporte deux chambres (8, 10) disposées de façon sensiblement parallèle, et espacées latéralement l'une de l'autre qui sont ouvertes dans des directions opposées, et dans lesquelles pénètrent les extrémités des barres (2, 5), et **en ce que** la seconde barre (2) est disposée dans la zone d'extrémité de sa chambre (10) de façon pivotante autour d'un axe (D) de façon que le mouvement de pivotement de la seconde barre (2) par rapport à la partie d'articulation formant pivot (3) soit limité par la chambre (10).

2. Articulation orthopédique selon la revendication 1, **caractérisée en ce que** la première barre (5) est engagée de façon rigide dans la chambre (8) correspondante.

3. Articulation orthopédique selon la revendication 1 ou 2, **caractérisée en ce que** la chambre (10) de la seconde barre (2) comprend une partie d'insert (11) guidée latéralement comportant des surfaces de butée (12, 13) pour le mouvement de pivotement de la seconde barre (2).

4. Articulation orthopédique selon la revendication 3, **caractérisée en ce que** la partie d'insert (11) est introduite de façon échangeable dans la partie d'articulation formant pivot (3).

5. Articulation orthopédique selon l'une des revendications 1 à 4, **caractérisée en ce que** la seconde barre (2) peut être poussée vers l'une des surfaces de butée (12, 13) au moyen d'un ressort (7).

6. Articulation orthopédique selon la revendication 5, **caractérisée en ce que** la surface supérieure de la partie d'articulation formant pivot (3) porte un bouton de fixation (6) en forme de champignon qui est entouré par une bande de caoutchouc élastique qui enlace avec tension la seconde barre (2).

7. Articulation orthopédique selon l'une des revendications 1 à 6, **caractérisée en ce qu'**elle est constituée par une orthèse du coup de pied comportant une partie de pied en tant que première partie d'articulation (4) et une partie de cheville en tant que seconde partie d'articulation (1).
